# EUROPÄISCHE PATENTANMELDUNG

(11) **EP 1 767 233 A1**
(43) Veröffentlichungstag der Anmeldung: **28.03.2007**
(21) Anmeldenummer: 06016681.6
(22) Anmeldetag: 10.08.2006
(51) Int. Cl.: A61M 5/145

(54) **Spritzenaufnahme für einen medizinischen Injektor**

(30) Priorität: 23.09.2005 DE 102005045391
(71) Anmelder: Medtron AG, 66128 Saarbrücken (DE)
(72) Erfinder: Koch, Wolfgang, 66584 Spiesen (DE); Altmeyer, Hanno, 66538 Neunkirchen (DE)
(74) Vertreter: Müller-Gerbes Wagner Albiger Patentanwälte

(57) **Zusammenfassung**

Die Erfindung betrifft eine Spritzenaufnahme für einen medizinischen Injektor, umfassend ein zylindrisches geschlossenes Aufnahmelager (1) mit einer Aufnahmebohrung (1a), in die das rückwärtige Ende einer Spritze einsteckbar ist, wobei die an der Spritze ausgebildeten Verriegelungselemente von quer zur Längsachse derselben nach außen vorstehenden Verriegelungsnocken gebildet sind, die im Bereich des rückwärtigen Endes der Spritze umfangsseitig vorstehen, wobei das Aufnahmelager mehrteilig mit einer Basisplatte (10), einem Haltering (11) und einem Lagerring (12) ausgebildet ist, die gemeinsam die Aufnahmebohrung begrenzen und als Verriegelungselemente Verriegelungslaschen (111) vorgesehen sind, die verschiebbar im Aufnahmelager angeordnet sind, dergestalt, dass sie aus einer Freigabeposition durch Verschiebung in eine Verriegelungsposition überführbar sind, in der sie mit den Verriegelungsnocken einer in die Aufnahmebohrung (1a) eingesteckten Spritze (2) ohne Drehung derselben um ihre Längsachse in Wirkverbindung bringbar sind, und umgekehrt.

## Beschreibung

Die Erfindung betrifft eine Spritzenaufnahme für einen medizinischen Injektor, umfassend ein zylindrisches geschlossenes Aufnahmelager mit einer Aufnahmebohrung, in die das rückwärtige Ende einer Spritze einsteckbar ist und mittels an der Spritze und dem Aufnahmelager ausgebildeten und in Wirkverbindung bringbaren Verriegelungselementen in der Aufnahmebohrung lösbar verriegelbar ist, wobei die an der Spritze ausgebildeten Verriegelungselemente von quer zur Längsachse derselben nach außen vorstehenden Verriegelungsnocken gebildet sind, die im Bereich des rückwärtigen Endes der Spritze umfangsseitig vorstehen.

Spritzenaufnahmen der eingangs genannten Art sind vielfältig bekannt und werden bei medizinischen Injektoren, beispielsweise in der Angiographie verwendet, um z. B. einem Patienten eine Kontrastmitteldosis zu verabreichen. Hierbei wird ein medizinischer Injektor verwendet, der mit einer Spritzenaufnahme ausgerüstet ist, in welche eine mit dem zu verabreichenden Fluid, beispielsweise einem Kontrastmittel gefüllte Spritze eingelegt und nachfolgend vom Injektor in den Patienten injiziert wird.

Aus der gattungsbildenden EP 0 587 567 B1 ist eine Spritzenaufnahme bekannt, bei der die Spritze mit ihrem rückwärtigen Ende voraus in eine Spritzenaufnahme eingelegt wird und über im Bereich des vorderen Endes der Spritze ausgebildete Verriegelungselemente mit der Spritzenaufnahme verriegelt wird, wobei zur Verriegelung eine Drehung der Spritze um ihre Längsachse erforderlich ist, um die Verriegelungselemente in Wirkverbindung zu bringen. Ebenso ist es zur Entnahme der Spritze aus der Spritzenaufnahme notwendig, die Spritze in entgegengesetzter Richtung um ihre Längsachse zu drehen, so dass die Verriegelungselemente wieder außer Eingriff kommen. Diese bei der bekannten Spritzenaufnahme erforderliche Drehung bzw. Rotation der Spritze um ihre Längsachse, um die Verriegelungselemente in Eingriff zu bringen, stellt einen unerwünscht hohen manuellen Aufwand dar und bringt überdies die Schwierigkeit mit sich, dass eine Kupplung zwischen der auf den Kolben der Spritze einwirkenden Kolbenstange und dem Kolben die Rotationsbewegung nachvollziehen muss, was nicht nur aufwändig, sondern auch störanfällig ist und von daher verbesserungswürdig erscheint.

Darüber hinaus ist speziell für den hier vorgesehenen Anwendungsbereich eine Spritze handelsüblich erhältlich, an deren rückwärtigen Ende umfangsseitig nach außen vorstehende Verriegelungsnocken vorgesehen sind, so dass diese Spritze eine Verriegelung an ihrem rückwärtigen Ende erfordert.

Aufgabe der vorliegenden Erfindung ist es daher, eine Spritzenaufnahme der eingangs genannten Art dahingehend weiterzubilden, dass sie eine einfache und schnell zu bewerkstelligende Verriegelung einer Spritze in ihrem rückwärtigen Bereich ohne erforderliche Rotation der Spritze um ihre eigene Achse ermöglicht.

Diese Aufgabe wird durch Ausbildung einer Spritzenaufnahme für einen medizinischen Injektor gemäß den Merkmalen des Patentanspruches 1 gelöst.

Vorteilhafte Ausgestaltungen und Weiterbildungen der Erfindung sind Gegenstand der Unteransprüche.

Der erfindungsgemäße Vorschlag beruht darauf, das Aufnahmelager mehrteilig mit einer Basisplatte, einem Haltering und einem Lagerring auszubilden, die gemeinsam die Aufnahmebohrung begrenzen und als Verriegelungselemente Verriegelungslaschen vorgesehen sind, die verschiebbar im Aufnahmelager angeordnet sind, dergestalt, dass sie aus einer Freigabeposition durch Verschiebung in eine Verriegelungsposition überführbar sind, in der sie mit den Verriegelungsnocken einer in die Aufnahmebohrung eingesteckten Spritze ohne Drehung derselben um ihre Längsachse in Wirkverbindung bringbar sind, und umgekehrt.

Gemäß einem Vorschlag der Erfindung sind hierbei die Verriegelungslaschen senkrecht zur Längsachse der eingesteckten Spritze verschiebbar.

Eine alternative Ausführungsform sieht vor, das Aufnahmelager mehrteilig mit einer Basisplatte, einem Haltering und einem Lagerring auszubilden, die gemeinsam die Aufnahmebohrung begrenzen und relativ zueinander um die Längsachse der Aufnahmebohrung drehbar sind, wobei zumindest ein Teil des Aufnahmelagers, vorzugsweise der Haltering, mit innenseitig vorstehenden Verriegelungslaschen als Verriegelungselement ausgebildet ist, die durch Verdrehung um die Längsachse der Aufnahmebohrung lösbar mit den Verriegelungsnocken einer in die Aufnahmebohrung eingesteckten Spritze in Wirkverbindung bringbar sind, so dass eine Drehung der Spritze um ihre Längsachse nicht notwendig ist.

Erfindungsgemäß ist es demnach in einer überraschend einfachen Weise möglich, eine Spritze von vorne, d. h. mit ihrem rückwärtigen Ende voran in die Aufnahmebohrung des Aufnahmelagers einzustecken, in dieser Position zu halten und sodann durch Drehung eines Teils der Aufnahmebohrung die Verriegelungselemente in Wirkverbindung mit den Verriegelungsnocken der Spritze zu bringen, so dass die Spritze im Aufnahmelager gesichert wird. In ebensolch einfacher Weise ist auch die Entnahme der Spritze in entsprechender kinematischer Umkehr ermöglicht.

Bevorzugt sind die Basisplatte und der Lagerring der erfindungsgemäßen Spritzenaufnahme starr miteinander verbunden und nicht drehbar gehaltert und der Haltering ist zwischen Basisplatte und Lagerring drehbar angeordnet und mit den innenseitig vorstehenden Verriegelungslaschen ausgebildet, um die Verriegelung der in die Aufnahmebohrung des Aufnahmelagers eingesetzten Spritze zu bewirken.

Hierbei wird es als vorteilhaft angesehen, wenn die Basisplatte und der Lagerring unter Zwischenlage des Halteringes miteinander verschraubt sind und der Lagerring auf seiner der Basisplatte zugewandten Seite mit einander diametral gegenüberliegenden Lagersegmenten ausgebildet ist, auf denen der Haltering drehbar gelagert ist. Es versteht sich jedoch, dass die Erfindung nicht auf diese Ausführungsform beschränkt ist, sondern es ist auch denkbar, die Lagersegmente an der Basisplatte auszubilden und den Lagerring auf die vorstehenden Lagersegmente der Basisplatte unter Zwischenlage des Halteringes aufzuschrauben.

Damit der auf den Lagersegmenten drehbar gelagerte Haltering exakt geführt ist und seine innenseitig vorstehenden Verriegelungslaschen beim Einstecken bzw. Herausziehen der Spritze aus der Aufnahmebohrung des Aufnahmelagers nicht stören, sind bevorzugt die Lagersegmente mit schlitzförmigen Ausnehmungen zur Aufnahme der Verriegelungslaschen des Halteringes ausgebildet, wenn diese nicht mit den Verriegelungsnocken in Wirkverbindung stehen, so dass sie zuverlässig außer Eingriff gebracht werden und die Bewegung der Spritze in bzw. aus der Aufnahmebohrung nicht behindern.

Um die Bedienbarkeit der erfindungsgemäßen Spritzenaufnahme weiter zu erleichtern und Fehlbedienungen vorzubeugen, ist die relative Verdrehbarkeit der Teile des Aufnahmelager zueinander auf einen Winkel von bevorzugt 90° begrenzt, so dass eine Verdrehbarkeit zwischen zwei Endpositionen gewährleistet wird. In der einen Endposition ist die vollständige Freigabe einer in die Aufnahmebohrung eingesteckten Spritze bewirkbar, während in der anderen Endposition die vollständige Verriegelung einer in die Aufnahmebohrung eingesetzten Spritze bewirkt wird. Somit ist es bei Handhabung der erfindungsgemäßen Spritzenaufnahme lediglich notwendig, die Spritze so weit wie möglich in die Aufnahmebohrung einzustecken und nachfolgend das bewegliche Teil, vorzugsweise den Haltering in seine die Verriegelung bewirkende Endposition zu verdrehen.

Um eine exakt lagerichtige Einführung des rückwärtigen Endes der Spritze in die Aufnahmebohrung des Aufnahmelagers der erfindungsgemäßen Spritzenaufnahme zu gewährleisten, ist die Spritze bevorzugt an ihrem rückwärtigen Ende mit Führungsnocken ausgerüstet und das Aufnahmelager ist entsprechend in seinen die Aufnahmebohrung umgebenden Wandbereichen mit Ausnehmungen für die Führungsnocken und die Verriegelungsnocken der Spritze ausgebildet, so dass diese nur in einer exakt definierten Lage in die Aufnahmebohrung eingeführt werden kann. Hierbei sind die Ausnehmungen bevorzugt im Lagerring des Aufnahmelagers der erfindungsgemäßen Spritzenaufnahme ausgebildet.

Um ein leichtes Betätigen des die Verriegelung bevorzugt bewirkenden drehbaren Halteringes zu gewährleisten, ist dieser nach einem weiteren Vorschlag der Erfindung mit einer außenseitigen Profilierung und/oder einem vorstehenden Betätigungshebel ausgebildet, um das Ergreifen und Verdrehen durch einen Bediener zu erleichtern.

Schließlich kann der Haltering an seinen stirnseitigen Enden, die den jeweils benachbarten weiteren Teilen des Aufnahmelagers, nämlich der Basisplatte einerseits und dem Lagerring andererseits zugewandt sind, mit umlaufenden Nuten zur Aufnahme von Dichtungsringen ausgerüstet sein, um einen eventuellen Austritt von Fluid aus der Spritze über das Aufnahmelager zu verhindern. Weitere Einzelheiten der Erfindung werden nachfolgend im Detail anhand der lediglich ein Ausführungsbeispiel darstellenden Zeichnung erläutert. Es zeigen:
- Figur 1: die erfindungsgemäße Spritzenaufnahme nebst Spritze in einer Explosionsdarstellung,
- Figur 2: die zusammengesetzte erfindungsgemäße Spritzenaufnahme nebst einsetzbarer Spritze in einer perspektivischen Darstellung,
- Figur 3: eine weitere Ausführungsform der Erfindung.

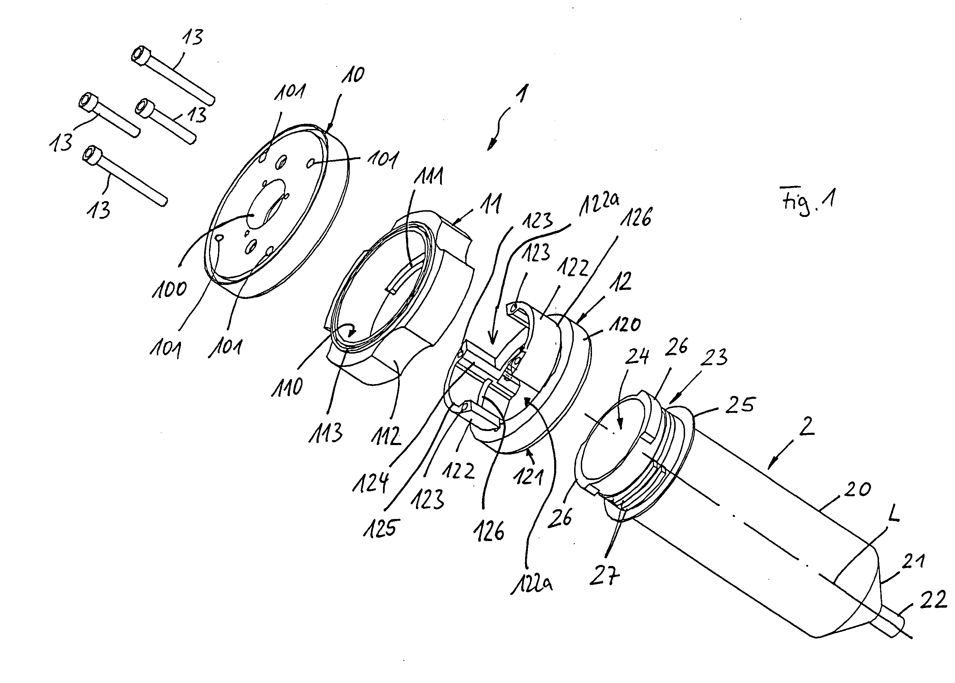

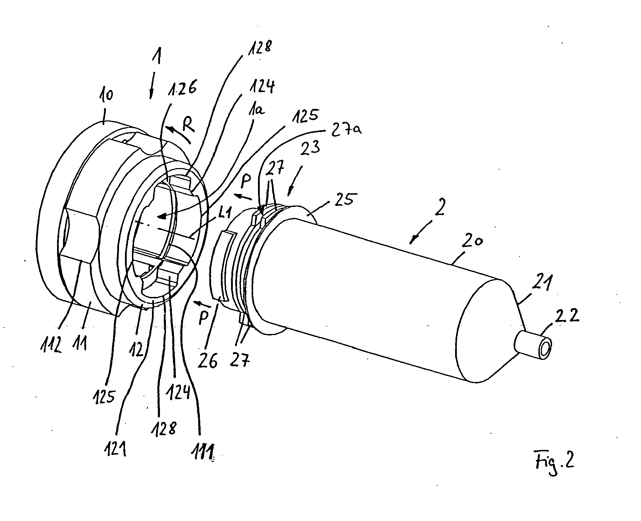

In den Figuren 1 und 2 ist eine mit Bezugsziffer 1 gekennzeichnete Spritzenaufnahme für einen medizinischen Injektor, der in seinen weiteren Einzelheiten nicht gezeigt ist, dargestellt, wobei diese Spritzenaufnahme 1 der Aufnahme einer insbesondere mit einem zu injizierenden Fluid vorgefüllten Spritze 2 dient. Als Fluid kann beispielsweise ein Kontrastmittel vorgesehen sein.

Die Spritze 2 umfasst einen zylindrischen Korpus 20 mit einem vorderen Ende 21 und dort angeordnetem Auslassstutzen 22 und einem rückwärtigen Ende 23, welches ferner eine zentrale Öffnung 24 aufweist, wobei im Innern des zylindrischen Korpus 20 in an sich bekannter, hier jedoch nicht dargestellter Weise, ein abdichtend an der Wandung geführter Kolben eingesetzt ist.

Im Bereich des rückwärtigen Endes 23 der Spritze 2 ist darüber hinaus ein umfangsseitig nach außen vorstehender umlaufender Anschlagvorsprung 25 ausgebildet, ferner sind benachbart zu diesem jeweils in diametral gegenüberliegenden Bereichen des Korpus 20 radial nach außen vorstehende und voneinander beabstandete Verriegelungsnocken 27 vorgesehen, zwischen denen jeweils ein Freiraum 27a definiert ist.

Unmittelbar benachbart zur Öffnung 24 sind an diametral gegenüberliegenden Bereichen und jeweils um 90° zu den Verriegelungsnocken 27 versetzt zwei radial nach außen vorstehende Führungsnocken 26 vorgesehen.

Demzufolge stehen sowohl die Führungsnocken 26 wie auch die Verriegelungsnocken 27 quer zur Längsachse L der Spritze 2 nach außen vor.

Um eine solchermaßen ausgebildete Spritze 2 in einem medizinischen Injektor für die automatisiert erfolgende Injektion des in der Spritze 2 bevorrateten Fluids einzusetzen, ist die Spritzenaufnahme 1 vorgesehen. Diese Spritzenaufnahme 1 befindet sich am vorderen Ende eines hier nicht dargestellten medizinischen Injektors und umfasst ein zylindrisches geschlossenes Aufnahmelager 1 mit einer Aufnahmebohrung 1 a, in die das rückwärtige Ende 23 der Spritze 2 einsteckbar ist. In nicht dargestellter Weise wird das Aufnahmelager 1 entlang der Längsachse L1 von einer Kolbenstange des Injektors durchsetzt, die sodann bei in die Spritzenaufnahme 1 eingesetzter Spritze den Antrieb des Kolbens innerhalb der Spritze 2 bewirkt.

Der Aufbau des Aufnahmelagers 1 ist insbesondere aus der Explosionsdarstellung gemäß Figur 1 ersichtlich.

Wie dieser Figur 1 im Vergleich zu dem Zusammenbauzustand des Aufnahmelagers 1 gemäß Figur 2 entnehmbar, wird das Aufnahmelager 1 von insgesamt drei Teilen gebildet, nämlich einer Basisplatte 10, einem Haltering 11 und einem Lagerring 12.

Die Basisplatte 10 wird auf der der Spritze 2 abgewandten Seite des Aufnahmelagers 1 angeordnet und über nicht näher dargestellte Verbindungsmittel mit den weiteren Teilen des Injektors verbunden. Die Basisplatte 10 weist eine zentrale Durchgangsbohrung 100 auf, durch welche die nicht dargestellte Kolbenstange des Injektors in Richtung auf den Kolben einer eingesetzten Spritze 2 hindurch tritt. Ferner weist die Basisplatte 10 parallel zur zentralen Durchgangsbohrung 100 verlaufende Durchgangsbohrungen 101 auf, durch welche Befestigungsschrauben 13 hindurch gesteckt werden können.

Am anderen Ende des Aufnahmelagers 1, d. h. der einzusetzenden Spritze 2 zugewandt, wird der Lagerring 12 vorgesehen. Dieser Lagerring 12 umfasst einen Ringkorpus 120, auf dessen der Basisplatte 10 zugewandten Seite zwei diametral in Bezug auf die Längsachse L1 einander gegenüberliegende und in Richtung auf die Basisplatte 10 vorstehende Lagersegmente 122 angeformt sind. Die Lagersegmente 122 weisen ausgehend von ihrer der Basisplatte 10 zugewandten Oberfläche Gewindebohrungen 123 auf, die beim Aufsetzen der Lagersegmente 122 auf die Basisplatte 10 mit den darin ausgebildeten Durchgangsbohrungen 101 fluchten, so dass eine Verschraubung von Basisplatte 10 und Lagerring 12 mittels der Befestigungsschrauben 13 bewirkbar ist und somit die Basisplatte 10 und der Lagerring 12 starr miteinander verbunden und vorzugsweise nicht drehbar am Injektor befestigt sind.

Das dritte Teil des Aufnahmelagers 1, nämlich der Haltering 11 wird zwischen dem Ringkorpus 120 des Lagerringes 12 und der Basisplatte 10 angeordnet, und zwar derart, dass der Haltering 11 mit seiner zylindrischen Innenoberfläche 110 auf den Außenoberflächen der beiden Lagersegmente 122 drehbar gelagert ist. Der Haltering 11 weist überdies zwei diametral einander gegenüberliegende und innenseitig über die zylindrische Innenoberfläche 110 vorstehende Verriegelungslaschen 111 auf. Beim Zusammenbau des Aufnahmelagers 1 gemäß Explosionsdarstellung in Figur 1 sind diese Verriegelungslaschen 111 im Bereich der Freiräume 122a zwischen den beiden einander gegenüberliegenden Lagersegmenten 122 des Lagerringes 12 angeordnet.

Um nun eine Verdrehung des Halteringes 11 relativ zu den starr aneinander befestigten weiteren Teilen des Aufnahmelagers 1, nämlich der Basisplatte 10 und des Lagerringes 12 zu ermöglichen, sind die Lagersegmente 122 ausgehend von ihren jeweiligen Freiräumen 122a mit schlitzförmigen Ausnehmungen 126 im Übergangsbereich zum Ringkorpus 120 ausgebildet, in welche bei Verdrehung des Halteringes 11 gemäß Pfeil R in Figur 2 die innenseitig vorstehenden Verriegelungslaschen 111 des Halteringes 11 aufgenommen werden. Durch entsprechende Längendimensionierung dieser schlitzförmigen Ausnehmungen 126 wird gewährleistet, dass der Haltering 11 um exakt 90° relativ zum Lagerring 12 und zur Basisplatte 10 um die Längsachse L1 des Aufnahmelagers 1 verdreht werden kann.

Darüber hinaus ist aus der Darstellung gemäß Figur 1 noch ersichtlich, dass die Gewindebohrungen 123 in den Lagersegmenten 122, welcher der Aufnahme der Befestigungsschrauben 13 dienen, jeweils von innenseitig vorstehenden Führungsstegen 124 begrenzt sind.

Wie sich nun aus einer vergleichenden Darstellung des Einbauzustandes gemäß Figur 2 ergibt, begrenzen diese innenseitig in die Aufnahmebohrung 1 a des Aufnahmelagers 1 vorstehenden Führungsstege 124 jeweils wechselweise Ausnehmungen 125 bzw. 128, die in den die Aufnahmebohrung 1a umgebenden Wandbereichen des Aufnahmelagers 1 im Bereich des Lagerringes 12 ausgebildet sind.

Die Ausnehmungen 125 sind hierbei den Führungsnocken 26 der Spritze 2 zugeordnet und entsprechend dimensioniert, während die Ausnehmungen 128 den Verriegelungsnocken 27 zugeordnet und entsprechend dimensioniert sind.

Von daher ist es möglich, die Spritze 2 mit ihrem rückwärtigen Ende 23 voran gemäß Pfeilen P in die Aufnahmebohrung 1a des im zusammengebauten Zustand zylindrischen geschlossenen Aufnahmelagers 1 einzuschieben, und zwar so weit, bis der umlaufende Anschlagvorsprung 25 der Spritze 2 am vorderen Rand 121 des Lagerringes 12 zur Anlage kommt. Während des Einschiebens der Spritze 2 ist der Haltering 11 in eine solche Position verdreht, dass seine innenseitig vorstehenden Verriegelungslaschen 111 in den schlitzförmigen Ausnehmungen 126 der Lagersegmente 122 angeordnet sind, d. h. sich innerhalb der entsprechenden Ausnehmungen 125 für die Führungsnocken 26 der Spritze befinden. Da die Führungsnocken 26 der Spritze eine geringere Höhe als die Verriegelungsnocken 27 aufweisen, ist das Einschieben des rückwärtigen Endes 23 der Spritze 2 in dieser Stellung ermöglicht.

Nachdem nun die Spritze 2 so weit gemäß Pfeilen P in die Aufnahmebohrung 1a des Aufnahmelagers 1 eingeschoben worden ist, dass der umlaufende Anschlagvorsprung 25 am vorderen Ende 121 des Aufnahmelagers 1 bzw. dessen Lagerring 12 anliegt, wird der Haltering 11 gemäß Pfeil R um 90° verdreht, so dass die innenseitig vorstehenden Verriegelungslaschen 111 aus den schlitzförmigen Ausnehmungen 126 der Lagersegmente 122 in den Bereich der Freiräume 122a vortreten und dort zwischen die in dieser Position befindlichen Verriegelungsnocken 27, d. h. in den zwischen diesen gebildeten Freiraum 27a eintreten, so dass die Verriegelungselemente, welche einerseits von den vorstehenden Verriegelungsnocken 27 und andererseits von den innenseitig vorstehenden Verriegelungslaschen 111 gebildet werden, miteinander in Wirkverbindung treten und eine Festlegung der Spritze 2 innerhalb des Aufnahmelagers 1 bewirkt ist, ohne dass es einer Rotation der Spritze 2 um ihre Längsachse L bedurfte.

Nunmehr kann in an sich bekannter Weise der Injektionsvorgang mittels des nicht dargestellten medizinischen Injektors erfolgen. Zur Entnahme der Spritze 2 aus dem Aufnahmelager 1 wird die zuvor erläuterte Kinematik exakt umgekehrt ausgeführt. Zunächst wird der Haltering 11 entgegen Pfeil R aus seiner verriegelnden Position um 90° gedreht, so dass die innenseitig vorstehenden Verriegelungslaschen 111 außer Eingriff mit den Verriegelungsnocken 27 kommen und wieder in den schlitzförmigen Ausnehmungen 126 der Lagersegmente 122 zum Liegen kommen. Nunmehr ist die Spritze 2 freigegeben und kann entgegen Pfeilen P geradlinig in Richtung der Längsachse L aus der Aufnahmebohrung 1a des Aufnahmelagers 1 entnommen werden.

Es ist offensichtlich, dass sowohl das Einsetzen wie auch das Entnehmen einer Spritze 2 in bzw. aus dem Aufnahmelager 1 von einer einzelnen Person mit zwei Handgriffen durchgeführt werden kann. Während diese Person mit der einen Hand die Spritze 2 hält und geradlinig gemäß Pfeilen P in die Aufnahmebohrung 1a einführt, kann sodann mit der zweiten Hand die Verdrehung des Halteringes 11 zur Verriegelung der Spritze 2 innerhalb der Aufnahmebohrung 1a vorgenommen werden und entsprechend umgekehrt bei der Entnahme der Spritze 2 aus der Aufnahmebohrung 1a.

Aufgrund der den jeweiligen Führungsnocken 26 bzw. Verriegelungsnocken 27 zugeordneten Ausnehmungen 125 bzw. 128 am Lagerring 12 ist nur eine lagerichtige und damit die Funktionsfähigkeit des Aufnahmelagers 1 sicherstellende definierte und lagerichtige Einführung der Spritze 2 ermöglicht, was Fehlbedienungen ausschließt.

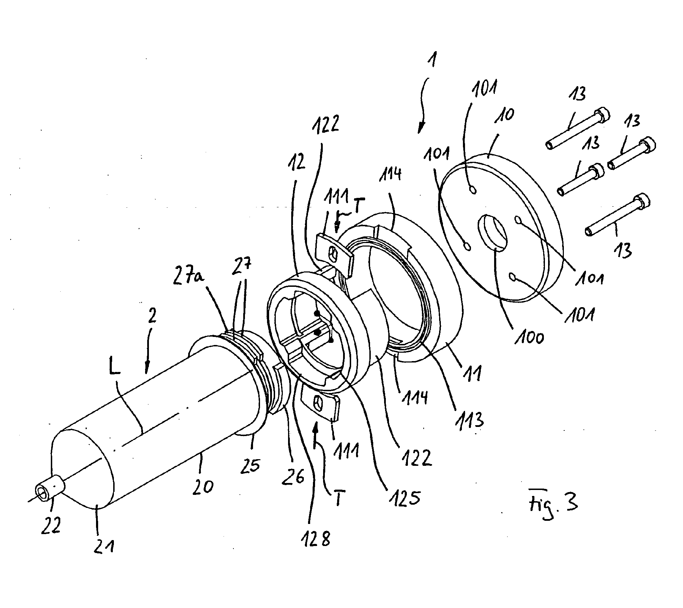

In der Figur 3 ist eine gegenüber dem vorangehend erläuterten Ausführungsbeispiel gemäß Figuren 1 und 2 abweichende Ausführungsform einer Spritzenaufnahme dargestellt, bei der gleiche Teile mit gleichen Bezugsziffern versehen sind und zur Vermeidung von Wiederholungen nicht nochmals gesondert erläutert werden, sofern dies nicht zum Verständnis dieser Ausführungsform erforderlich ist.

Das sich aus den drei Teilen, nämlich Basisplatte 10, Haltering 11 und Lagerring 12 zusammensetzende Aufnahmelager 1 wird mittels der Schrauben 13 zusammengefügt, so dass eine Spritze 2 mit ihrem rückwärtigen Ende voran in die zylindrische Aufnahmebohrung einsteckbar ist.

Zur Verriegelung der Spritze 2 in dieser eingesteckten Position im Aufnahmelager 1 ist beim Ausführungsbeispiel gemäß Figur 3 der Haltering 11 nicht drehbar auf dem Lagerring 12 angeordnet, sondern starr mit diesem verbunden. Der Haltering 11 weist jedoch an diametral gegenüberliegenden Bereichen, die den Ausnehmungen 128 im Lagerring 12 zur Aufnahme der Verriegelungsnocken 27 der Spritze 2 zugeordnet sind, zwei Ausnehmungen 114 auf, die sodann mit den Freiräumen zwischen den beiden Lagersegmenten 122 korrespondieren. In diese Ausnehmungen 114 sind Verriegelungslaschen 111 einsteckbar, so dass diese Verriegelungslaschen in Pfeilrichtung T senkrecht zur Längsachse L der eingesteckten Spritze verschiebbar sind.

Demgemäß können die Verriegelungslaschen 111 aus einer ersten, die Verriegelungsnocken 27 und den zwischen diesen ausgebildeten Freiraum 27a freigebenden Position nach Einstecken der Spritze durch Verschieben in Pfeilrichtung T in eine solche Position vorgeschoben werden, in der sie in den Freiraum 27a zwischen zwei benachbarten Verriegelungsnocken 27 eintreten und somit mit den Verriegelungsnocken 27 in Wirkverbindung treten, so dass die Spritze 2 nicht aus dem Aufnahmelager 1 entnommen werden kann.

Zum Entnehmen der nicht mehr benötigten Spritze 2 aus dem Aufnahmelager 1 werden analog die Verriegelungslaschen 111 entgegen Pfeilen T verschoben, so dass sie außer Eingriff mit den Verriegelungsnocken 27 der Spritze gelangen und von daher die Spritze 2 aus der Aufnahmebohrung des Aufnahmelagers 1 entnommen werden kann.

Auch bei dieser alternativen Ausführungsform des Aufnahmelagers 1 gemäß Figur 3 ist von daher zur Verriegelung bzw. Freigabe der Spritze 2 im bzw. aus dem Aufnahmelager 1 keine Drehung der Spritze 2 um ihre Längsachse L erforderlich.

## Patentansprüche

1. Spritzenaufnahme für einen medizinischen Injektor, umfassend ein zylindrisches geschlossenes Aufnahmelager (1) mit einer Aufnahmebohrung (1a), in die das rückwärtige Ende (23) einer Spritze (2) einsteckbar ist und mittels an der Spritze (2) und dem Aufnahmelager (1) ausgebildeten und in Wirkverbindung bringbaren Verbindungselementen in der Aufnahmebohrung (1a) lösbar verriegelbar ist, wobei die an der Spritze (2) ausgebildeten Verriegelungselemente von quer zur Längsachse (L) derselben nach außen vorstehenden Verriegelungsnocken (27) gebildet sind, die im Bereich des rückwärtigen Endes (23) der Spritze (2) umfangsseitig vorstehen, **dadurch gekennzeichnet, dass** das Aufnahmelager (1) mehrteilig mit einer Basisplatte (10), einem Haltering (11) und einem Lagerring (12) ausgebildet ist, die gemeinsam die Aufnahmebohrung (1a) begrenzen und als Verriegelungselemente Verriegelungslaschen (111) vorgesehen sind, die verschiebbar im Aufnahmelager (1) angeordnet sind, dergestalt, dass sie aus einer Freigabeposition durch Verschiebung in eine Verriegelungsposition überführbar sind, in der sie mit den Verriegelungsnocken (27) einer in die Aufnahmebohrung (1a) eingesteckten Spritze (2) ohne Drehung derselben um ihre Längsachse (L) in Wirkverbindung bringbar sind, und umgekehrt.

2. Spritzenaufnahme nach Anspruch 1, **dadurch gekennzeichnet, dass** die Verriegelungslaschen rechtwinklig zur Längsachse (L) der Spritze (2) verschiebbar sind.

3. Spritzenaufnahme nach Anspruch 1, **dadurch gekennzeichnet, dass** die Basisplatte (10), der Haltering (11) und der Lagerring (12) relativ zueinander um die Längsachse (L) der Aufnahmebohrung (1a) drehbar sind und zumindest ein Teil (10, 11, 12) des Aufnahmelagers (1) mit innenseitig vorstehenden Verriegelungslaschen (111) ausgebildet ist, die durch Verdrehung um die Längsachse (L) der Aufnahmebohrung (1a) lösbar mit den Verriegelungsnocken (27) einer in die Aufnahmebohrung (1a) eingesteckten Spritze (2) ohne Drehung derselben um ihre Längsachse (L) in Wirkverbindung bringbar sind.

4. Spritzenaufnahme nach Anspruch 3, **dadurch gekennzeichnet, dass** die Basisplatte (10) und der Lagerring (12) starr miteinander verbunden sind und der Haltering zwischen Basisplatte (10) und Lagerring (12) drehbar angeordnet und mit den innenseitig vorstehenden Verriegelungslaschen (111) ausgebildet ist.

5. Spritzenaufnahme nach Anspruch 3 oder 4, **dadurch gekennzeichnet, dass** die Basisplatte (10) und der Lagerring (12) unter Zwischenlage des Halteringes (11) miteinander verschraubt sind und der Lagerring (12) auf seiner der Basisplatte (10) zugewandten Seite mit einander diametral gegenüberliegenden Lagersegmenten (122) ausgebildet ist, auf denen der Haltering (11) drehbar gelagert ist.

6. Spritzenaufnahme nach Anspruch 5, **dadurch gekennzeichnet, dass** die Lagersegmente (122) mit schlitzförmigen Ausnehmungen (126) zur Aufnahme der Verriegelungslaschen (111) des Halteringes (11) ausgebildet sind, wenn diese nicht mit den Verriegelungsnocken (27) in Wirkverbindung stehen.

7. Spritzenaufnahme nach einem der Ansprüche 3 bis 6, **dadurch gekennzeichnet, dass** die relative Verdrehbarkeit der Teile (10, 11, 12) des Aufnahmelagers (1) zueinander auf einen Winkel von 90° begrenzt ist.

8. Spritzenaufnahme nach einem der Ansprüche 1 bis 7, **dadurch gekennzeichnet, dass** die Spritze (2) an ihrem rückwärtigen Ende (23) mit Führungsnocken (26) ausgerüstet ist und das Aufnahmelager (1) in seinen die Aufnahmebohrung (1a) umgebenden Wandbereichen mit Ausnehmungen (125, 128) für die Führungsnocken (26) und die Verriegelungsnocken (27) der Spritze (2) ausgebildet ist.

9. Spritzenaufnahme nach Anspruch 8, **dadurch gekennzeichnet, dass** die Ausnehmungen (125, 128) für die Führungsnocken (26) und die Verriegelungsnocken (27) der Spritze (2) im Lagerring (12) ausgebildet sind.

10. Spritzenaufnahme nach einem der Ansprüche 1 bis 9, **dadurch gekennzeichnet, dass** der Haltering (11) mit einer außenseitigen Profilierung (112) und/oder einem vorstehenden Betätigungshebel ausgebildet ist.

11. Spritzenaufnahme nach einem der Ansprüche 1 bis 10, **dadurch gekennzeichnet, dass** der Haltering (11) an seinen stirnseitigen Enden mit umlaufenden Nuten (113) zur Aufnahme von Dichtungsringen ausgerüstet ist.
